# EUROPÄISCHE PATENTANMELDUNG

(11) **EP 0 761 662 A1**
(43) Veröffentlichungstag der Anmeldung: **12.03.1997**
(21) Anmeldenummer: 96113241.2
(22) Anmeldetag: 19.08.1996
(51) Int. Cl.: C07D 307/28, C07D 309/18, C07D 319/12, B01J 23/889, B01J 23/89

(54) **Verfahren zur Herstellung von ungesättigten cyclischen Ethern**

(30) Priorität: 23.08.1995 DE 19530993
(71) Anmelder: BASF AKTIENGESELLSCHAFT, 67056 Ludwigshafen (DE)
(72) Erfinder: Schröder, Jürgen, Dr., 67071 Ludwigshafen (DE); Fetzer, Thomas, Dr., 67346 Speyer (DE); Rieker, Christopher William, Dr., 68163 Mannheim (DE); Ebel, Klaus, Dr., 68623 Lampertheim (DE)

(57) **Zusammenfassung**

Verfahren zur Herstellung von ungesättigten cyclische Ethern der allgemeinen Formel I 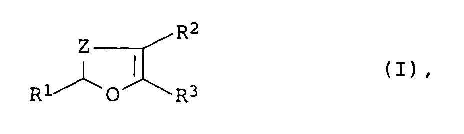 in der
- Z: -(CHR⁴)_{q}- oder -(CHR⁴)_{q}-O-
- q: 0, 1, 2 oder 3 und
- R¹,R²,R³,R⁴: Wasserstoff oder C₁- bis C₄-Alkyl
bedeuten, aus Diolen der allgemeinen Formel II 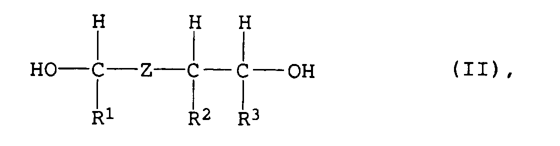 in der Z, R¹, R² und R³ die obengenannten Bedeutungen haben, in Gegenwart eines cobalthaltigen Trägerkatalysators, indem die cobalthaltigen Trägerkatalysatoren Cobalt und ein Edelmetall - aus der Gruppe Platin, Palladium, Rhodium, Iridium, Ruthenium, Osmium, Rhenium oder deren Gemische - im Atomverhältnis von 0,5:1 bis 70000:1 auf einem inerten Träger und gegebenenfalls basische Alkali-, Erdalkalimetallsalze, Scandium, Vanadin, Chrom, Mangan, Eisen, Nickel, Kupfer, Zink, Germanium, Zinn, Blei, Antimon, Wismut oder deren Gemische enthalten und man die Umsetzung in flüssiger Phase bei Temperaturen von 150 bis 300°C durchführt, sowie neue cobalthaltigen Trägerkatalysatoren, insbesondere hergestellt durch Soltränkung.

## Beschreibung

Die vorliegende Erfindung betrifft ein Verfahren zur Herstellung von ungesättigten cyclischen Ethern aus Diolen in flüssiger Phase an cobalthaltigen Trägerkatalysatoren sowie neue cobalthaltige Trägerkatalysatoren, insbesondere hergestellt durch Soltränkung.

Aus der DE-A-23 46 943 ist ein Verfahren zur Herstellung ungesättigter cyclischer Verbindungen aus Diolen unter einem Wasserstoffstrom bekannt, in dem als Katalysatoren Gemische aus einem Kupferchromit- oder Kupferträgerkatalysator und einer Wolfram- oder Heteropolywolframsäure eingesetzt werden. Die Umsätze und Ausbeuten lassen zu wünschen übrig.

Aus der JP-01/287079 ist ein Verfahren zur Herstellung von Dihydrofuranen und Dihydropyranen aus 1,4-Butandiolen bzw. 1,5-Pentandiolen an Cobaltkatalysatoren, die bei 600°C mit Wasserstoff reduziert werden müssen, bekannt.

Aus der US-A-2 993 910 ist ein Verfahren zur Herstellung von Dihydrofuranen aus 1,4-Butandiolen an Cobaltkatalysatoren, die bei 300 bis 450°C mit Wasserstoff reduziert werden müssen, bekannt.

Der vorliegenden Erfindung lag die Aufgabe zugrunde den vorgenannten Nachteilen abzuhelfen.

Demgemäß wurde ein neues und verbessertes Verfahren zur Herstellung von ungesättigten cyclische Ethern der allgemeinen Formel I 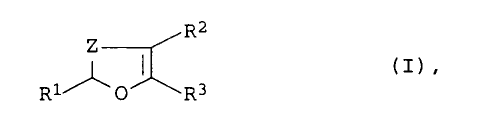 in der
- Z: -(CHR⁴)_{q}- oder -(CHR⁴)_{q}-O-
- q: 0, 1, 2 oder 3 und
- R¹,R²,R³,R⁴: Wasserstoff oder C₁- bis C₄-Alkyl
bedeuten, aus Diolen der allgemeinen Formel II 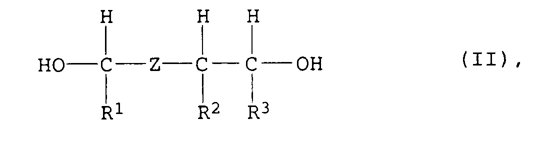 in der Z, R¹, R² und R³ die obengenannten Bedeutungen haben, in Gegenwart eines cobalthaltigen Trägerkatalysators, gefunden, welches dadurch gekennzeichnet ist, daß die cobalthaltigen Trägerkatalysatoren Cobalt und ein Edelmetall - aus der Gruppe Platin, Palladium, Rhodium, Iridium, Ruthenium, Osmium, Rhenium oder deren Gemische - im Atomverhältnis von 0,5:1 bis 70000:1 auf einem inerten Träger und gegebenenfalls basische Alkali-, Erdalkalimetallsalze, Scandium, Vanadin, Chrom, Mangan, Eisen, Nickel, Kupfer, Zink, Germanium, Zinn, Blei, Antimon, Wismut oder deren Gemische enthalten und man die Umsetzung in flüssiger Phase bei Temperaturen von 150 bis 300°C durchführt, sowie neue cobalthaltigen Trägerkatalysatoren, insbesondere hergestellt durch Soltränkung.

Das erfindungsgemäße Verfahren läßt sich wie folgt durchführen:

Man kann das Diol II in der Regel mit 1 bis 20 Gew.-%, bevorzugt 2 bis 10 Gew.-% cobalthaltigem Trägerkatalysator bei Temperaturen von 150 bis 300°C, bevorzugt 160 bis 240°C umsetzen und das entstandene Gemisch aus dem ungesättigten cyclischen Ether I und dem Reaktionswasser diskontinuierlich, bevorzugt kontinuierlich abdestillieren. Gegebenenfalls kann der bei der Reaktion entstehende ungesättigte cyclische Ether mit unter Reaktionsbedingungen inerten Gasen, wie Stickstoff oder Argon, gestrippt werden. Bei kontinuierlicher Fahrweise kann durch Zufuhr von frischem Diol II der Flüssigkeitsstand im Reaktionsgefäß gehalten werden. Um den Gehalt an destillativ nur schwer vom ungesättigten cyclischen Ether abtrennbaren gesättigten cyclischen Ether zu senken, können 0,05 bis 1 Gew.-%, bevorzugt 0,1 bis 0,3 Gew.-% Alkali- und/oder Erdalkalimetallverbindungen, z.B. als Hydroxid oder Carbonat, in den Reaktor dosiert werden.

Als cobalthaltige Trägerkatalysatoren eignen sich die Oxide des Cobalts oder metallisches Cobalt auf einem porösen Träger und ein oder mehrere Elemente der Edelmetalle aus der Gruppe Platin, Palladium, Rhodium, Iridium, Ruthenium, Osmium, Rhenium oder deren Gemische, bevorzugt Platin, Palladium, Rhenium oder deren Gemische, besonders bevorzugt Platin, Palladium oder deren Gemische und gegebenenfalls 0,01 bis 10 Gew.-%, bevorzugt 0,1 bis 5 Gew.-%, besonders bevorzugt 0,5 bis 3 Gew.-% basische Alkali-, Erdalkalimetallsalze, Scandium, Vanadin, Chrom, Mangan, Eisen, Nickel, Kupfer, Zink, Germanium, Zinn, Blei, Antimon, Wismut oder deren Gemische (Verbindung A), bevorzugt Lithium, Kalium, Natrium, Calcium, Strontium, Barium, Mangan, Eisen, Nickel, Kupfer, Zink, Zinn, Antimon oder deren Gemische, besonders bevorzugt Kalium, Natrium, Mangan, Eisen, Nickel, Kupfer, Zink oder deren Gemische enthalten.

Der Gewichtsanteil des Cobalt(-oxides) im Trägerkatalysator liegt in der Regel zwischen 1 und 70 Gew.-%, bevorzugt zwischen 5 und 50 Gew.-%, besonders bevorzugt zwischen 10 und 40 Gew.-%.

Zur Erhöhung der Aktivität kann dem Katalysator ein oder mehrere Elemente aus der Gruppe Platin, Palladium, Rhodium, Iridium, Ruthenium, Osmium oder Rhenium, bevorzugt Platin, Palladium oder Rhenium, besonders bevorzugt Platin oder Palladium zugegeben werden.

Der Gewichtsanteil dieser Elemente liegt zwischen 0,001 und 2 Gew.-%, bevorzugt zwischen 0,005 und 1 Gew.-%, besonders bevorzugt zwischen 0,01 und 0,5 Gew.-%.

Die Trägerkatalysatoren haben in der Regel ein Gewichtsverhältnis von Cobalt zum Edelmetall von 0,5:1 bis 70.000:1, bevorzugt 5:1 bis 10000:1, besonders bevorzugt 20:1 bis 4000:1.

Als Träger eignen sich inerte Träger wie SiO₂, Al₂O₃, TiO₂, ZrO₂, Zeolithe aller Art wie kleinporige Zeolithe, z.B. A-Zeolith, mittelporige Zeolithe, z.B. ZSM-5, ZSM-11, Ferrierit, großporige Zeolithe, z.B. Faujasite, β-Zeolithe, Mordenit, Offretit, hydrothermal hergestellte Phosphate wie AlPO und SAPO, Aktivkohlen oder Erdalkalioxide, bevorzugt SiO₂, ZrO₂ und Zeolithe, besonders bevorzugt SiO₂.

Die Trägerkatalysatoren weisen in der Regel eine BET-Oberfläche von 1 bis 600 m²/g, bevorzugt 10 bis 500 m²/g, besonders bevorzugt 50 bis 400 m²/g auf.

Die Porosität der Trägerkatalysatoren liegt in der Regel bei 0,01 bis 1,5 ml/g, bevorzugt 0,1 bis 1,2 ml/g, besonders bevorzugt 0,2 bis 1 ml/g.

Die Herstellung der Trägerkatalysatoren ist allgemein bekannt. Vorteilhaft ist die Tränkung des porösen Trägermateriales mit einer löslichen Cobaltverbindung (z.B. eines Nitrits, Nitrats, Sulfits, Sulfats, Carbonats, Hydroxids, von Carboxylaten, Halogeniden, Halogeniten, Halogenaten u.a.), gegebenenfalls gleichzeitig oder nacheinander mit einer ebenfalls löslichen Verbindung A (z.B. als Nitrit, Nitrat, Sulfit, Sulfat, Carbonat, Hydroxid, Carboxylate, Halogenide, Halogenite, Halogenate u.a.), und anschließender thermischer Zersetzung des Anions zum Oxid. Eine weitere Möglichkeit ist das Mischen einer Cobalt-Verbindung mit dem Trägermaterial (trocken oder in Suspension, insbesondere durch Sprühtrocknung), gegebenenfalls gleichzeitig mit einer chemischen Verbindung A, Verdichtung des Materials (z.B. durch Verkneten, gegebenenfalls Zugabe eines geeigneten Verformungshilfsmittels), Formgebung durch Extrudieren, Trocknung und anschließender Calcinierung bei Temperaturen von 200 bis 1300°C, bevorzugt 300 bis 1000°C, besonders bevorzugt 400 bis 800°C.

Das Element A kann auch nachträglich aufgetränkt, aufgesprüht oder sonstwie aufgebracht werden.

Eine oder mehrere der Edelmetallkomponenten können durch Tränkung mit einer löslichen Verbindung (Nitrit, Nitrat, Sulfit, Sulfat, Phosphit, Phosphat, Carbonat, Hydroxid, Carboxylate, Halogenide, Halogenite, Halogenate u.a.) oder als Säuren aufgebracht werden. Besonders bevorzugt ist die Aufbringung eines oder mehrerer dieser Metalle als Sol.

Die Substituenten R¹, R², R³, R⁴, das Zwischenglied Z und der Index q in den Verbindungen I und II haben folgende Bedeutungen:

R¹,R²,R³,R⁴ unabhängig voneinander
- Wasserstoff,
- C₁- bis C₄-Alkyl wie Methyl, Ethyl, n-Propyl, iso-Propyl, n-Butyl, iso-Butyl, sec.-Butyl und tert.-Butyl, bevorzugt C₁- bis C₃-Alkyl wie Methyl, Ethyl, n-Propyl und iso-Propyl, besonders bevorzugt Methyl und Ethyl,

Z
- -(CHR⁴)_{q}- oder -(CHR⁴)_{q}-O-,

q
- 0, 1, 2 oder 3, bevorzugt 0 und 1, besonders bevorzugt 1.

Als Diole II eignen sich z.B. 1,4-Butandiol, 1,5-Pentandiol, 1,6-Hexandiol, Diethylenglykol.

Als ungesättigte cyclische Ether I eignen sich z.B. 3,4-Dihydro-2H-pyrane, 2,3-Dihydrofuran und 1,4-Dioxan, bevorzugt 3,4-Dihydro-2H-pyran.

Die ungesättigten cyclischen Ether I sind wertvolle Schutzgruppen für Alkohole.

### Beispiele

### Herstellung der Katalysatoren A bis E

555 ml einer Lösung aus 291,3 g Co(NO₃)₂ · 6 H₂O und einem Metallnitrat in Wasser wurde mit 300 g SiO₂-Pulver (Wasseraufnahme 1,85 ml/g) ca. 2 h verrührt, 16 h bei 120°C getrocknet und 2 h bei 500°C calciniert.

Auf einer aus Tabelle 1 ersichtlichen Menge dieser Masse wurde innerhalb von 2 h ein Edelmetall-Sols (0,6 g/l), hergestellt durch Mischen eines Edelmetallsalzes in 700 ml Wasser mit 5 g Polyvinylpyrrolidon und 300 ml Ethanol und 4 h Erhitzen unter Rückfluß, aufgesprüht, anschließend getrocknet und 1 h bei 500°C calciniert.

Weitere Einzelheiten zur Herstellung und zu den Eigenschaften der Katalysatoren sind Tabelle 1 zu entnehmen:

**Tabelle 1**

| Katalysator | Edelmetallsalz [g] | Menge Co-halt. SiO₂ [g] | Menge Edelmetall-Sol [ml] | Edelmetallgehalt [Gew.-%] | BET-Oberfläche [m²/g] |
|---|---|---|---|---|---|
| A | 1,46 g Pt(NO₃)₂ | 310 | 260 | 0,05 | 219 |
| B | 1,46 g Pt(NO₃)₂ | 328,6 | 548 | 0,1 | 215 |
| C | 1,3 g Pt(NO₃)₂ | 332 | 277 | 0,05 | 221 |
| D | 2,92 g Ru(NO)(NO₃)₃ | 332 | 277 | 0,05 | 241 |
| E | 1,172 g ReCl₅ | 335 | 280 | 0,05 | 194 |

### Herstellung der Katalysatoren F bis H

555 ml einer Lösung aus 291,3 g Co(NO₃)₂ · 6 H₂O und einem Metallnitrat in Wasser wurde mit 300 g SiO₂-Pulver (Wasseraufnahme 1,85 ml/g) ca. 2 h verrührt, 16 h bei 120°C getrocknet und 2 h bei 500°C calciniert.

Auf einer aus Tabelle 2 ersichtlichen Menge dieser Masse auf einem Drehteller wurde innerhalb von 2 h 275 ml eines Edelmetall-Sols (0,6 g/l), hergestellt durch Mischen eines Edelmetallsalzes in 700 ml Wasser mit 5 g Polyvinylpyrrolidon und 300 ml Ethanol und 4 h Erhitzen unter Rückfluß, aufgesprüht, anschließend getrocknet und 1 h bei 500°C calciniert.

Weitere Einzelheiten zur Herstellung und zu den Eigenschaften der Katalysatoren sind Tabelle 2 zu entnehmen:

**Tabelle 2**

| Katalysator | Metallnitrat [g] | Edelmetallsalz [g] | Menge Co-halt. SiO₂ [g] | Menge Edelmetall-Sol [ml] | Edelmetallgehalt [Gew.-%] | BET-Oberfläche [m²/g] |
|---|---|---|---|---|---|---|
| F | 0,98 g NaNO₃ | 1,46 g Pt(NO₃)₂ | 330,1 | 275 | 0,05 | 231 |
| G | 1,11 g KNO₃ | 1,46 g Pt(NO₃)₂ | 340,5 | 284 | 0,05 | 198 |
| H | 11,4 g Cu(NO₃)₂ · 3 H₂O | 1,46 g Pt(NO₃)₂ | 308 | 257 | 0,05 | 350 |

### Herstellung des Katalysators J

555 ml einer Lösung aus 291,3 g Co(NO₃)₂ · 6 H₂O und einem Metallnitrat in Wasser wurde mit 300 g SiO₂-Pulver (Wasseraufnahme 1,85 ml/g) ca. 2 h verrührt, 16 h bei 120°C getrocknet und 2 h bei 500°C calciniert.

Auf 336,4 g der zuvor hergestellten Masse auf einem Drehteller wurde innerhalb von 2 h 250 ml einer wäßrigen, 0,41 g Pt(NO₃)₂ enthaltenden Lösung aufgesprüht, anschließend getrocknet und 1 h bei 500°C calciniert. Der entstandene Katalysator J enthielt 0,05 Gew.-% Platin und hatte eine BET-Oberfläche von 211 m²/g.

### Vergleichskatalysator X (nach US-A-2 993 910)

740 ml einer Lösung aus 388,4 g Co(NO₃)₂ · 6 H₂O in Wasser wurde mit 400 g SiO₂-Pulver (Wasseraufnahme 1,85 ml/g) ca. 2 h verrührt, 16 h bei 120°C getrocknet und 2 h bei 500°C calciniert (BET-Oberfläche: 230 m²/g).

### Beispiele 1 bis 9

1,4 Liter 1,5-Pentandiol und die aus Tabelle 3 ersichtliche Menge eines Katalysators A bis J wurden vorgelegt und unter Rühren bis zur unteren Grenze des Temperaturintervalls erwärmt und das entstehende 3,4-Dihydro-2H-pyran/Wasser-Gemisch kontinuierlich abdestilliert. Innerhalb der aus Tabelle 3 ersichtlichen Zeiten wurde kontinuierlich insgesamt die aus Tabelle 3 ersichtliche Menge 1,5-Pentandiol zudosiert. Nach Phasentrennung des Destillats erhielt man 3,4-Dihydro-2H-pyran in den in Tabelle 3 angegebenen Mengen und Spezifikationen.

**Tabelle 3**

| Beispiel Nr. | Katalysator | Temperatur [°C] | Dauer [h] | Gesamtmenge 1,5-Pentandiol [g] | Ausbeute 3,4-Dihydro-2H-pyran [g/%] | Reinheit [Gew.-%] | Gehalt an Tetrahydropyran [Gew.-%] |
|---|---|---|---|---|---|---|---|
| 1 | 45 g A | 180 - 215 | 200 | 10950 | 8498/98 | 97 | 1,5 |
| 2 | 45 g B | 180 - 215 | 200 | 11112 | 8639/98 | 96 | 1,3 |
| 3* | 30 g C | 190 - 235 | 100 | 5562 | 4144/94 | 95 | 1,3 |
| 4 | 30 g D | 190 - 235 | 50 | 2240 | 1715/97 | 96 | 2 |
| 5 | 45 g E | 180 - 230 | 100 | 5500 | 4300/99 | 98 | 1 |
| 6 | 45 g F | 185 - 225 | 200 | 11109 | 8613/98 | 95 | 1,8 |
| 7 | 45 g G | 185 - 235 | 200 | 10830 | 8361/98 | 96 | 1,3 |
| 8 | 45 g H | 160 - 215 | 200 | 10008 | 7671/97 | 95 | 1,4 |
| 9 | 45 g J | 180 - 215 | 200 | 10946 | 8513/98 | 96 | 1,6 |
| V1** | 45 g J | 185 - 240 | 200 | 8090 | 5897/92 | 91 | 6,7 |

| | | | | | | | |
|---|---|---|---|---|---|---|---|
| * Zusatz von 10 ml einer 1%igen wäßrigen Kaliumcarbonatlösung | | | | | | | |
| ** Vergleichsbeispiel (nach US-A-2 993 910) | | | | | | | |

## Patentansprüche

1. Verfahren zur Herstellung von ungesättigten cyclische Ethern der allgemeinen Formel I 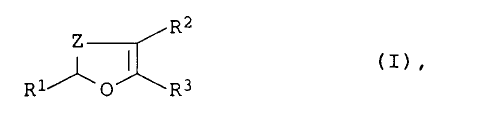 in der
Z -(CHR⁴)_{q}- oder -(CHR⁴)_{q}-O-
q 0, 1, 2 oder 3 und
R¹,R²,R³,R⁴ Wasserstoff oder C₁- bis C₄-Alkyl
bedeuten, aus Diolen der allgemeinen Formel II 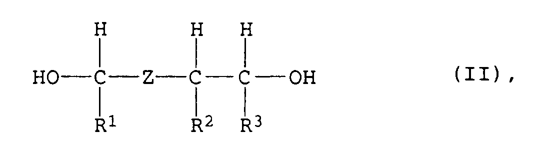 in der Z, R¹, R² und R³ die obengenannten Bedeutungen haben, in Gegenwart eines cobalthaltigen Trägerkatalysators, dadurch gekennzeichnet, daß die cobalthaltigen Trägerkatalysatoren Cobalt und ein Edelmetall - aus der Gruppe Platin, Palladium, Rhodium, Iridium, Ruthenium, Osmium, Rhenium oder deren Gemische - im Atomverhältnis von 0,5:1 bis 70000:1 auf einem inerten Träger und gegebenenfalls basische Alkali-, Erdalkalimetallsalze, Scandium, Vanadin, Chrom, Mangan, Eisen, Nickel, Kupfer, Zink, Germanium, Zinn, Blei, Antimon, Wismut oder deren Gemische enthalten und man die Umsetzung in flüssiger Phase bei Temperaturen von 150 bis 300°C durchführt.

2. Verfahren zur Herstellung von ungesättigten cyclische Ethern I nach Anspruch 1, dadurch gekennzeichnet, daß die cobalthaltigen Trägerkatalysatoren 1 bis 70 Gew.-% Cobalt und 0,001 bis 2 Gew.-% ein oder mehrere Edelmetalle enthalten.

3. Verfahren zur Herstellung von ungesättigten cyclische Ethern I nach den Ansprüchen 1 bis 2, dadurch gekennzeichnet, daß die cobalthaltigen Trägerkatalysatoren 0,001 bis 10 Gew.-% basische Alkali-, Erdalkalimetallsalze oder deren Gemische bezogen auf den Gesamtmetallgehalt enthalten.

4. Verfahren zur Herstellung von ungesättigten cyclische Ethern I nach den Ansprüchen 1 bis 3, dadurch gekennzeichnet, daß man während der Umsetzung 0,05 bis 1 Gew.-% basische Alkali-, Erdalkalimetallsalze oder deren Gemische bezogen auf I zusetzt.

5. Verfahren zur Herstellung von ungesättigten cyclische Ethern I nach den Ansprüchen 1 bis 4, dadurch gekennzeichnet, daß die cobalthaltigen Trägerkatalysatoren 0,01 bis 10 Gew.-% Scandium, Vanadin, Chrom, Mangan, Eisen, Nickel, Kupfer, Zink, Germanium, Zinn, Blei, Antimon, Wismut oder deren Gemische bezogen auf den Gesamtmetallgehalt enthalten.

6. Verfahren zur Herstellung von ungesättigten cyclische Ethern I nach den Ansprüchen 1 bis 5, dadurch gekennzeichnet, daß man die Elemente Platin, Palladium, Rhodium, Iridium, Ruthenium, Osmium, Rhenium oder deren Gemische als Sol auf die cobalthaltigen Trägerkatalysatoren aufbringt.

7. Cobalthaltige Trägerkatalysatoren bestehend aus Cobalt und einem Edelmetall - aus der Gruppe Platin, Palladium, Rhodium, Iridium, Ruthenium, Osmium, Rhenium oder deren Gemische - im Atomverhältnis von 0,5:1 bis 70000:1 auf einem inerten Träger und gegebenenfalls basische Alkali-, Erdalkalimetallsalze, Scandium, Vanadin, Chrom, Mangan, Eisen, Nikkel, Kupfer, Zink, Germanium, Zinn, Blei, Antimon, Wismut oder deren Gemischen.

8. Cobalthaltige Trägerkatalysatoren, hergestellt durch Soltränkung, bestehend aus Cobalt und einem Edelmetall - aus der Gruppe Platin, Palladium, Rhodium, Iridium, Ruthenium, Osmium, Rhenium oder deren Gemische - im Atomverhältnis von 0,5:1 bis 70000:1 auf einem inerten Träger und gegebenenfalls basische Alkali-, Erdalkalimetallsalze, Scandium, Vanadin, Chrom, Mangan, Eisen, Nickel, Kupfer, Zink, Germanium, Zinn, Blei, Antimon, Wismut oder deren Gemischen.
